# EUROPEAN PATENT APPLICATION

(11) **EP 4 276 727 A1**
(43) Date of publication of application: **15.11.2023**
(21) Application number: 22893969.0
(22) Date of filing: 12.12.2022
(51) Int. Cl.: G06Q 30/06, G06Q 30/02, H04L 9/32, H04L 67/53, G06Q 50/10, G06Q 50/00, H04L 9/00

(54) **METHOD AND SERVER FOR MANAGING DESIGN INFORMATION ON TOBACCO-RELATED ARTICLE**

(30) Priority: 30.03.2022 KR 20220039596
(71) Applicant: KT & G Corporation, Daejeon 34337 (KR)
(72) Inventor: JANG, Yong Joon, Daejeon 34128 (KR)
(74) Representative: Ter Meer Steinmeister & Partner
(86) International application number: PCT/KR2022/020130
(87) International publication number: WO 2023/191244

(57) **Abstract**

According to an embodiment, to manage design information of a tobacco-related article, it is possible to receive target type information of a tobacco-related target article and target design information of the target article from a seller terminal, issue a blockchain-based non-fungible token (NFT) containing ownership information indicating a seller of the seller terminal is an initial owner of the target design information, perform a purchase contract of the NFT between the seller and an administrator of the server, and store ownership change information of the NFT when the purchase contract is performed.

## Description

### Technical Field

The following embodiments relate to a technique for managing design information of a tobacco-related article.

### Background Art

In the past, designing tobacco-related articles was the unique task of a company selling tobacco and its partners. A method for direct dealing between a general consumer or a design seller and a tobacco company is needed to utilize designs highly preferred by general consumers for tobacco-related articles.

NFT stands for a non-fungible token, and an NFT is based on a blockchain network and characterized by assigning a separate and unique identification value to a digital asset such as a game or an artwork. Relevant information, such as ownership, changes in ownership, and sales history of an article assigned with an NFT, is all stored in a blockchain.

The above description is information the inventor(s) acquired during the course of conceiving the present disclosure, or already possessed at the time, and is not necessarily art publicly known before the present application was filed.

### Disclosure of the Invention

### Technical Goals

An embodiment may provide a method of managing design information of a tobacco-related article.

An embodiment may provide a server for performing a method of managing design information of a tobacco-related article.

### Technical Solutions

According to an embodiment, there is provided a method of managing design information of a tobacco-related article, performed by a server, the method including receiving target type information of a tobacco-related target article and target design information of the target article from a seller terminal, issuing a blockchain-based non-fungible token (NFT) containing ownership information indicating a seller of the seller terminal is an initial owner of the target design information, performing a purchase contract of the NFT between the seller and an administrator of the server, and storing ownership change information of the NFT when the purchase contract is performed.

The method may further include storing the target design information of the target article based on the type information of the target article, receiving the target type information of the target article from a user terminal, and outputting one or more pieces of design information associated with the target type information to the user terminal.

The method may further include receiving the target type information of the target article from the seller terminal, and providing basic design information of the target article to the seller terminal based on the target type information.

The method may further include providing a follow function between users of the server, and providing follow rankings of the users to a user terminal based on the follow function.

The method may further include providing a reward to the seller when the user terminal selects the target design information.

According to an embodiment, there is provided a server for performing a method of managing design information of a tobacco-related article, the server including a memory configured to store a program for managing design information, and a processor configured to execute the program, wherein the processor may be configured to perform receiving target type information of a tobacco-related target article and target design information of the target article from a seller terminal, issuing a blockchain-based NFT containing ownership information indicating a seller of the seller terminal is an initial owner of the target design information, performing a purchase contract of the NFT between the seller and an administrator of the server, and storing ownership change information of the NFT when the purchase contract is performed.

The program may be further configured to perform storing the target design information of the target article based on the type information of the target article, receiving the target type information of the target article from a user terminal, and outputting one or more pieces of design information associated with the target type information to the user terminal.

The program may be further configured to perform receiving the target type information of the target article from the seller terminal, and providing basic design information of the target article to the seller terminal based on the target type information.

The program may be further configured to perform providing a follow function between users of the server, and providing follow rankings of the users to a user terminal based on the follow function.

The program may be further configured to perform providing a reward to the seller when the user terminal selects the target design information.

### Effects

It is possible to provide a method of managing design information of a tobacco-related article.

It is possible to provide a server for performing a method of managing design information of a tobacco-related article.

### Brief Description of Drawings

FIG. 1 illustrates a system for managing design information of a tobacco-related article according to an embodiment.
FIGS. 2 to 4 are diagrams illustrating examples of a cigarette inserted into an aerosol generating device according to an example.
FIGS. 5 and 6 are views of examples of a cigarette according to an example.
FIG. 7 is a block diagram illustrating an aerosol generating device according to another example.
FIG. 8 is a block diagram illustrating an aerosol generating device according to still another example.
FIG. 9 is a diagram illustrating a configuration of a server according to an embodiment.
FIG. 10 is a flowchart illustrating a method of managing design information of a tobacco-related article according to an embodiment.
FIG. 11 is a flowchart illustrating a method of providing design information to a user according to an example.
FIG. 12 is a flowchart illustrating a method of providing basic design information to a seller according to an example.
FIG. 13 is a flowchart illustrating a method of selecting design information based on a follow function and providing a reward according to an example.
FIG. 14 is a flowchart illustrating a method of selecting design information by a user according to an example.

### Best Mode for Carrying Out the Invention

The following detailed structural or functional description is provided as an example only and various alterations and modifications may be made to the examples. Here, the embodiments are not construed as limited to the disclosure and should be understood to include all changes, equivalents, and replacements within the idea and the technical scope of the disclosure.

Terms, such as first, second, and the like, may be used herein to describe components. Each of these terminologies is not used to define an essence, order or sequence of a corresponding component but used merely to distinguish the corresponding component from other component(s). For example, a first component may be referred to as a second component, and similarly, the second component may also be referred to as the first component.

It should be noted that if it is described that one component is "connected", "coupled", or "joined" to another component, a third component may be "connected", "coupled", and "joined" between the first and second components, although the first component may be directly connected, coupled, or joined to the second component.

The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or populations thereof.

Unless otherwise defined, all terms, including technical and scientific terms, used herein have the same meaning as commonly understood by those having ordinary skill in the art to which this disclosure pertains. Terms, such as those defined in commonly used dictionaries, are to be interpreted as having a meaning that is consistent with their meaning in the context of the relevant art, and are not to be interpreted in an idealized or overly formal sense unless expressly so defined herein.

Hereinafter, embodiments will be described in detail with reference to the accompanying drawings. When describing the embodiments with reference to the accompanying drawings, like reference numerals refer to like elements and a repeated description related thereto will be omitted.

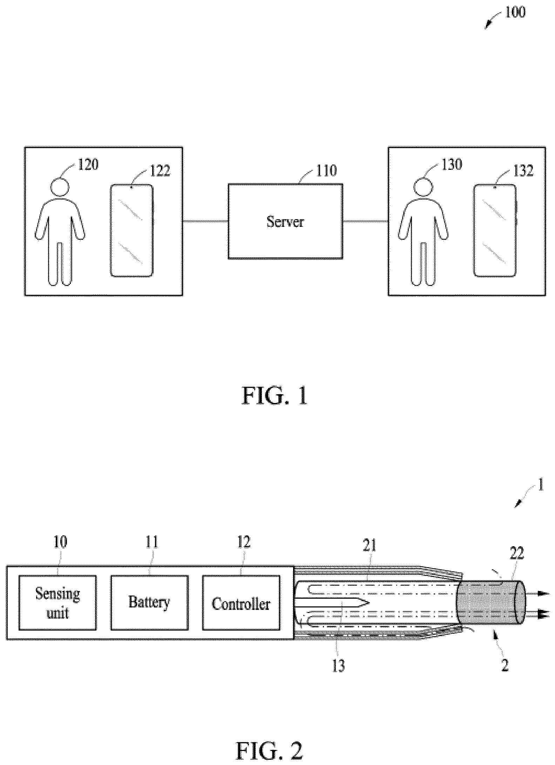

FIG. 1 illustrates a system for managing design information of a tobacco-related article according to an embodiment.

According to an embodiment, a system 100 may include a server 110, a first user terminal 122 of a seller 120, and a second user terminal 132 of a user 130. For example, the system 100 may include the first user terminal 122 of the seller 120 and the server 110. As another example, the system 100 may include the server 110 and the second user terminal 132 of the user 130. Hereinafter, the term "first user terminal" and the term "seller terminal" may be used and understood interchangeably.

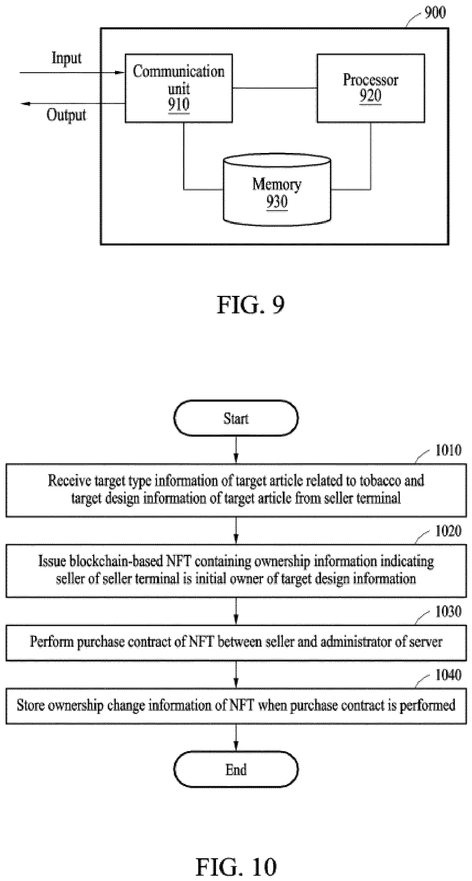

According to an embodiment, an administrator (not shown) of the server 110 may be a company that sells tobacco or tobacco-related articles. The seller 120 may be a user who transmits (or uploads) a design to the server 110 through the first user terminal 122. The user 130 may be a general user who accesses the server 110 through the first user terminal 132. The server 110 will be described in detail below with reference to FIG. 9.

According to one aspect, the user terminals 122 and 132 may be typical mobile communication devices such as smartphones.

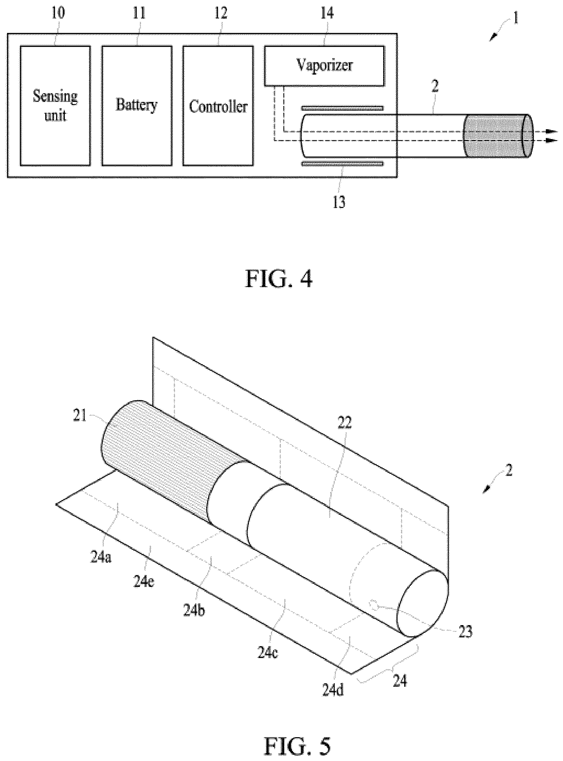

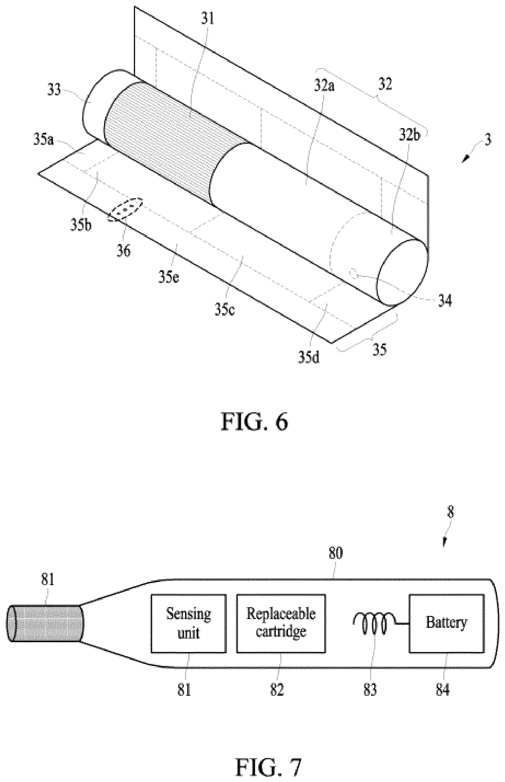

The seller 120 may create a design for a tobacco-related article. For example, the tobacco-related article may be the aerosol generating device 1, 8, 9 of FIGS. 2 to 4, 7, and 8 or the cigarette 2, 3 of FIGS. 5 and 6. As another example, the tobacco-related article may be a wrapper for general stick-type cigarettes, a waterpipe, a hand-rolled cigarette, a cigar, or a cigarillo. As still another example, the tobacco-related article may be a cartridge for an aerosol generating device or a cigarette-type electronic cigarette device. The tobacco-related article is not limited to the described embodiments.

For example, a design for the tobacco-related article may be a design for the shape of the article. As another example, the design for the tobacco-related article may be a graphic design visually shown on the outside of the article.

According to an embodiment, when the seller 120 wants to sell a design for a tobacco-related article, the seller 120 may need to create an account and a wallet of a web (or application) provided by the server 110. For example, the wallet may be a virtual wallet that contains information on the present condition of possession of a predetermined virtual currency or cash for trading a non-fungible token (hereinafter, "NFT") of the seller 120. When a trade for a design occurs, the seller 120 may check his/her own sales history through the wallet.

According to an embodiment, the user 130 may access the web (or application) provided by the server 110 through the second user terminal 132 and view various designs uploaded to the server 110. For example, the designs uploaded to the server 110 may be designs with ownership held by the seller 120 or purchased by the administrator of the server 110 from the seller 120.

To use various functions including a follow function provided by the server 110, the user 130 may need to create an account and a wallet of the web (or application) provided by the server 110 in advance.

FIGS. 2 to 4 are diagrams illustrating examples of a cigarette inserted into an aerosol generating device according to an example.

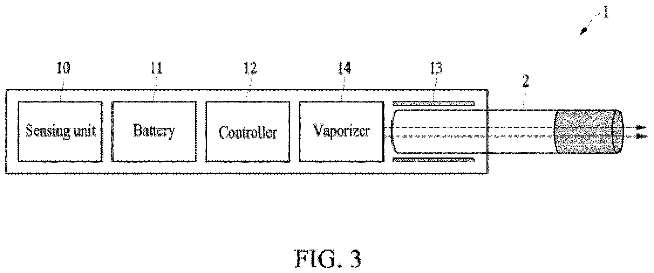

Referring to FIG. 2, an aerosol generating device 1 may include a sensing unit 10, a battery 11, a controller 12, and a heater 13. Referring to FIGS. 3 and 4, the aerosol generating device 1 may further include a vaporizer 14. Further, a cigarette 2 may be inserted into an inner space of the aerosol generating device 1. The aerosol generating device 1 may be owned by the seller 120 or the user 130 described above with reference to FIG. 1.

The aerosol generating device 1 shown in FIGS. 2 to 4 may include components related to the embodiments described herein. Therefore, it is to be understood by those having ordinary skill in the art to which the present disclosure pertains that the aerosol generating device 1 may further include other generally used components in addition to the ones shown in FIGS. 2 to 4.

In addition, although it is shown that the heater 13 is included in the aerosol generating device 1 in FIGS. 3 and 4, the heater 13 may be omitted as needed.

FIG. 2 illustrates a linear alignment of the sensing unit 10, the battery 11, the controller 12, and the heater 13. In addition, FIG. 3 illustrates a linear alignment of the battery 11, the controller 12, the vaporizer 14, and the heater 13. Further, FIG. 4 illustrates a parallel alignment of the vaporizer 14 and the heater 13. However, the internal structure of the aerosol generating device 1 is not limited to what is shown in FIGS. 2 to 4. That is, such alignments of the sensing unit 10, the battery 11, the controller 12, the heater 13, and the vaporizer 14 may be changed depending on the design of the aerosol generating device 1.

When the cigarette 2 is inserted into the aerosol generating device 1, the aerosol generating device 1 may actuate the heater 13 and/or the vaporizer 14 to generate an aerosol. The aerosol generated by the heater 13 and/or the vaporizer 14 may pass through the cigarette 2 into the user.

Even when the cigarette 2 is not inserted in the aerosol generating device 1, the aerosol generating device 1 may heat the heater 13, as needed.

The battery 11 may supply power to be used to operate the aerosol generating device 1. For example, the battery 11 may supply power to heat the heater 13 or the vaporizer 14, and may supply power required for the controller 12 to operate. In addition, the battery 11 may supply power required to operate a display, a sensor, a motor, or the like installed in the aerosol generating device 1.

The controller 12 may control the overall operation of the aerosol generating device 1. For example, the controller 12 may control respective operations of other components included in the aerosol generating device 1, in addition to the sensing unit 10, the battery 11, the heater 13, and the vaporizer 14. In addition, the controller 12 may verify a state of each of the components of the aerosol generating device 1 to determine whether the aerosol generating device 1 is in an operable state.

The controller 12 may include at least one processor. The at least one processor may be implemented as an array of a plurality of logic gates, or may be implemented as a combination of a general-purpose microprocessor and a memory in which a program executable by the microprocessor is stored. In addition, it is to be understood by those having ordinary skill in the art to which the disclosure pertains that the at least one processor may be implemented in other types of hardware.

The heater 13 may be heated by the power supplied by the battery 11. For example, when the cigarette is inserted in the aerosol generating device 1, the heater 13 may be disposed outside the cigarette. The heated heater 13 may thus raise the temperature of an aerosol generating material in the cigarette.

The heater 13 may be an electrically resistive heater. In this example, the heater 13 may include an electrically conductive track, and the heater 13 may be heated as a current flows through the electrically conductive track. However, the heater 13 is not limited to the foregoing example, and any example of heating the heater 13 up to a desired temperature may be applicable without limitation. Here, the desired temperature may be preset in the aerosol generating device 1 or may be set by the user.

For another example, the heater 13 may be an induction heater. Specifically, the heater 13 may include an electrically conductive coil for heating the cigarette in an induction heating manner, and the cigarette may include a susceptor to be heated by the induction heater.

For example, the heater 13 may include a tubular heating element, a plate-shaped heating element, a needle-shaped heating element, or a rod-shaped heating element, and may heat the inside or outside of the cigarette 2 according to the shape of a heating element.

In addition, the heater 13 may be provided as a plurality of heaters in the aerosol generating device 1. In this case, the heaters 13 may be disposed to be inserted into the cigarette 2, or may be disposed outside the cigarette 2. In addition, some of the heaters 13 may be disposed to be inserted into the cigarette 2, and the rest may be disposed outside the cigarette 2. However, the shape of the heater 13 is not limited to what is shown in FIGS. 2 to 4 but may be provided in various shapes.

The vaporizer 14 may heat a liquid composition to generate an aerosol, and the generated aerosol may pass through the cigarette 2 into the user. That is, the aerosol generated by the vaporizer 14 may travel along an airflow path of the aerosol generating device 1, and the airflow path may be configured such that the aerosol generated by the vaporizer 14 passes through the cigarette 2 into the user.

For example, the vaporizer 14 may include a liquid storage, a liquid transfer means, and a heating element. However, embodiments are not limited thereto. For example, the liquid storage, the liquid transfer means, and the heating element may be included as independent modules in the aerosol generating device 1.

The liquid storage may store the liquid composition. The liquid composition may be, for example, a liquid including a tobacco-containing material that includes a volatile tobacco flavor component, or may be a liquid including a non-tobacco material. The liquid storage may be manufactured to be detachable and attachable from and to the vaporizer 14, or may be manufactured in an integral form with the vaporizer 14.

The liquid composition may include, for example, water, a solvent, ethanol, a plant extract, a fragrance, a flavoring agent, or a vitamin mixture. The fragrance may include, for example, menthol, peppermint, spearmint oil, various fruit flavors, and the like. However, embodiments are not limited thereto. The flavoring agent may include ingredients that provide the user with a variety of flavors or scents. The vitamin mixture may be a mixture of at least one of vitamin A, vitamin B, vitamin C, or vitamin E. However, embodiments are not limited thereto. The liquid composition may also include an aerosol former such as glycerin and propylene glycol.

The liquid transfer means may transfer the liquid composition in the liquid storage to the heating element. The liquid transfer means may be, for example, a wick such as cotton fiber, ceramic fiber, glass fiber, or porous ceramic. However, embodiments are not limited thereto.

The heating element may be an element for heating the liquid composition transferred by the liquid transfer means. The heating element may be, for example, a metal heating wire, a metal heating plate, a ceramic heater, or the like. However, embodiments are not limited thereto. In addition, the heating element may include a conductive filament such as a nichrome wire, and may be arranged in a structure wound around the liquid transfer means. The heating element may be heated as a current is supplied and may transfer heat to the liquid composition in contact with the heating element, and may thereby heat the liquid composition. As a result, an aerosol may be generated.

For example, the vaporizer 14 may also be referred to as a cartomizer or an atomizer. However, embodiments are not limited thereto.

Meanwhile, the aerosol generating device 1 may further include general-purpose components in addition to the sensing unit 10, the battery 11, the controller 12, the heater 13, and the vaporizer 14. For example, the aerosol generating device 1 may include a display that outputs visual information and/or a motor that outputs tactile information.

According to an embodiment, the sensing unit 10 may include one or more biosensors. For example, the biosensors may include one or more of a blood pressure sensor, an electrocardiogram sensor, or a blood oxygen saturation sensor. The biosensors may include sensors configured to measure biosignals of the user, and are not limited to the embodiments described above. When the user grabs the aerosol generating device 1, a biosignal of the user may be measured.

According to an embodiment, the sensing unit 10 may include a sensor configured to measure a body composition of the user. For example, the body composition may include a skeletal muscle mass, a basal metabolic rate, a body water content, and a body fat mass. When the user grabs the aerosol generating device 1, the body composition of the user may be measured.

According to an embodiment, the sensing unit 10 may further include a puff detection sensor, a temperature detection sensor, a cigarette insertion detection sensor. In addition, the aerosol generating device 1 may be manufactured to have a structure in which external air may be introduced or internal gas may flow out even with the cigarette 2 being inserted.

Although not shown in FIGS. 2 to 4, the aerosol generating device 1 may constitute a system along with a separate cradle. For example, the cradle may be used to charge the battery 11 of the aerosol generating device 1. Alternatively, the cradle may be used to heat the heater 13, with the cradle and the aerosol generating device 1 coupled.

The cigarette 2 may be of a similar type to a general burning type. For example, the cigarette 2 may be divided into a first portion including an aerosol generating material and a second portion including a filter or the like. Alternatively, the second portion of the cigarette 2 may also include the aerosol generating material. For example, the aerosol generating material provided in the form of granules or capsules may be inserted into the second portion.

The first portion may be entirely inserted into the aerosol generating device 1, and the second portion may be exposed outside. Alternatively, the first portion may be partially inserted into the aerosol generating device 1, and the first portion may be entirely inserted and the second portion may be partially inserted into the aerosol generating device 1. The user may then inhale an aerosol with the second portion in their mouth. In this case, an aerosol may be generated as external air passes through the first portion, and the generated aerosol may pass through the second portion into the mouth of the user.

For example, the external air may be introduced through at least one air path formed in the aerosol generating device 1. In this example, the opening or closing and/or the size of the air path formed in the aerosol generating device 1 may be adjusted by the user. Accordingly, an amount of atomization, a sense of smoking, or the like may be adjusted by the user. As another example, the external air may be introduced into the inside of the cigarette 2 through at least one hole formed on a surface of the cigarette 2.

Hereinafter, examples of the cigarette 2 will be described with reference to FIGS. 5 and 6.

FIGS. 5 and 6 are views of examples of a cigarette according to an example.

Referring to FIG. 5, the cigarette 2 may include a tobacco rod 21 and a filter rod 22. The first portion 21 and the second portion 22 described above with reference to FIGS. 2 to 4 may include the tobacco rod 21 and the filter rod 22, respectively.

Although the filter rod 22 is illustrated as having a single segment in FIG. 5, embodiments are not limited thereto. That is, the filter rod 22 may include a plurality of segments. For example, the filter rod 22 may include a segment that cools an aerosol and a segment that filters out certain components contained in an aerosol. In addition, the filter rod 22 may further include at least one segment that performs another function, as needed.

The diameter of the cigarette 2 may be in a range of 5 millimeters (mm) to 9 mm, and the length thereof may be about 48 mm. However, embodiments are not limited thereto. For example, the length of the tobacco rod 21 may be about 12 mm, the length of a first segment of the filter rod 22 may be about 10 mm, the length of a second segment of the filter rod 22 may be about 14 mm, and the length of a third segment of the filter rod 22 may be about 12 mm. However, embodiments are not limited thereto.

The cigarette 2 may be wrapped with at least one wrapper 24. The wrapper 24 may have at least one hole through which external air is introduced or internal gas is discharged outside. For example, the cigarette 2 may be wrapped with one wrapper 24. As another example, the cigarette 2 may be wrapped with two or more wrappers 24 in an overlapping manner. For example, the tobacco rod 21 may be wrapped with a first wrapper 24a, and the filter rod 22 may be wrapped with wrappers 24b, 24c, and 24d. In addition, the cigarette 2 may be entirely wrapped again with a single wrapper 24e. For example, when the filter rod 22 includes a plurality of segments, the segments may be wrapped with the wrappers 24b, 24c, and 24d, respectively.

The first wrapper 24a and the second wrapper 24b may be formed of general filter wrapping paper. For example, the first wrapper 24a and the second wrapper 24b may be porous wrapping paper or non-porous wrapping paper. In addition, the first wrapper 24a and the second wrapper 24b may be formed of oilproof paper and/or an aluminum laminated wrapping material.

The third wrapper 24c may be formed of hard wrapping paper. For example, the basis weight of the third wrapper 24c may be in a range of 88 grams per square meter (g/m²) to 96 g/m², and may be desirably in a range of 90 g/m² to 94 g/m². In addition, the thickness of the third wrapper 24c may be in a range of 120 micrometers (µm) to 130 µm, and may be desirably 125 µm.

The fourth wrapper 24d may be formed of oilproof hard wrapping paper. For example, the basis weight of the fourth wrapper 24d may be in a range of 88 g/m² to 96 g/m², and may be desirably in a range of 90 g/m² to 94 g/m². In addition, the thickness of the fourth wrapper 24d may be in a range of 120 µm to 130 µm, and may be desirably 125 µm.

The fifth wrapper 24e may be formed of sterile paper (e.g., MFW). Here, the sterile paper (MFW) may refer to paper specially prepared such that it has enhanced tensile strength, water resistance, smoothness, or the like, compared to general paper. For example, the basis weight of the fifth wrapper 24e may be in a range of 57 g/m² to 63 g/m², and may be desirably 60 g/m². In addition, the thickness of the fifth wrapper 24e may be in a range of 64 µm to 70 µm, and may be desirably 67 µm.

The fifth wrapper 24e may have a predetermined material internally added thereto. The material may be, for example, silicon. However, embodiments are not limited thereto. Silicon may have properties, such as, for example, heat resistance which is characterized by less change by temperature, oxidation resistance which refers to resistance to oxidation, resistance to various chemicals, water repellency against water, or electrical insulation. However, silicon may not be necessarily used, but any material having such properties described above may be applied (or coated) to the fifth wrapper 24e without limitation.

The fifth wrapper 24e may prevent the cigarette 2 from burning. For example, there may be a probability that the cigarette 2 burns when the tobacco rod 21 is heated by the heater 13. For example, when the temperature rises above an ignition point of any one of materials included in the tobacco rod 21, the cigarette 2 may burn. Even in this case, it may still be possible to prevent the cigarette 2 from burning because the fifth wrapper 24e includes a non-combustible material.

In addition, the fifth wrapper 24e may prevent a holder from being contaminated by substances produced in the cigarette 2. For example, liquid substances may be produced in the cigarette 2 by puffs from the user. For example, as an aerosol generated in the cigarette 2 is cooled by external air, such liquid substances (e.g., water, etc.) may be produced. Thus, wrapping the cigarette 2 with the fifth wrapper 24e may prevent the liquid substances produced in the cigarette 2 from leaking out of the cigarette 2.

Although the wrappers 24a, 24b, 24c, 24d, and 24e have been described with reference to FIG. 5, the materials or shapes of the wrappers 24a, 24b, 24c, 24d, and 24e are not limited to the described embodiment, and may include various materials or shapes surrounding a medium (e.g., the tobacco rod 21) or a filter rod (e.g., the filter rod 22).

The tobacco rod 21 may include an aerosol generating material. The aerosol generating material may include, for example, at least one of glycerin, propylene glycol, ethylene glycol, dipropylene glycol, diethylene glycol, triethylene glycol, tetraethylene glycol, or oleyl alcohol. However, embodiments are not limited thereto. The tobacco rod 21 may also include other additives, such as, for example, a flavoring agent, a wetting agent, and/or an organic acid. In addition, the tobacco rod 21 may include a flavoring liquid such as menthol or a moisturizing agent that is added as being sprayed onto the tobacco rod 21.

The tobacco rod 21 may be manufactured in various forms. For example, the tobacco rod 21 may be manufactured as a sheet or as a strand. The tobacco rod 21 may also be formed with a cut tobacco filler from finely cut tobacco sheets. In addition, the tobacco rod 21 may be enveloped by a heat-conductive material. The heat-conductive material may be, for example, a metal foil such as an aluminum foil. However, embodiments are not limited thereto. For example, the heat-conductive material enveloping the tobacco rod 21 may evenly distribute the heat transferred to the tobacco rod 21 to improve the thermal conductivity to be applied to the tobacco rod 21, thereby improving the taste of tobacco. In addition, the thermally conductive material enveloping the tobacco rod 21 may function as a susceptor heated by an induction heater. In this case, although not shown, the tobacco rod 21 may further include an additional susceptor in addition to the thermally conductive material enveloping the outside thereof.

The filter rod 22 may be a cellulose acetate filter. However, there is no limit to the shape of the filter rod 22. For example, the filter rod 22 may be a cylindrical rod, or a tubular rod including a hollow therein. The filter rod 22 may also be a recess-type rod. For example, when the filter rod 22 includes a plurality of segments, at least one of the segments may be manufactured in a different shape.

A first segment of the filter rod 22 may be a cellulose acetate filter. For example, the first segment may be a tubular structure including a hollow therein. The first segment may prevent internal materials of the tobacco rod 21 from being pushed back when the heater 13 is inserted and generate an aerosol cooling effect. A desirable diameter of the hollow included in the first segment may be adopted from a range of 2 mm to 4.5 mm. However, embodiments are not limited thereto.

A desirable length of the first segment may be adopted from a range of 4 mm to 30 mm. However, embodiments are not limited thereto. The length of the first segment may be desirably 10 mm. However, embodiments are not limited thereto.

The first segment may have a hardness that is adjustable through an adjustment of the content of a plasticizer in a process of manufacturing the first segment. In addition, the first segment may be manufactured by inserting a structure such as a film or a tube of the same or different materials inside (e.g., the hollow).

A second segment of the filter rod 22 may cool an aerosol generated as the heater 13 heats the tobacco rod 21. The user may thus inhale the aerosol cooled down to a suitable temperature.

The length or diameter of the second segment may be determined in various ways according to the shape of the cigarette 2. For example, a desirable length of the second segment may be adopted from a range of 7 mm to 20 mm. The length of the second segment may be desirably about 14 mm. However, embodiments are not limited thereto.

The second segment may be manufactured by weaving a polymer fiber. In this case, a flavoring liquid may be applied to fiber formed of a polymer. Alternatively, the second segment may be manufactured by weaving a separate fiber to which a flavoring liquid is applied and the fiber formed of the polymer together. Alternatively, the second segment may be formed with a crimped polymer sheet.

For example, the polymer may be prepared with a material selected from the group consisting of polyethylene (PE), polypropylene (PP), polyvinyl chloride (PVC), polyethylene terephthalate (PET), polylactic acid (PLA), cellulose acetate (CA,) and aluminum foil.

As the second segment is formed with the woven polymer fiber or the crimped polymer sheet, the second segment may include a single channel or a plurality of channels extending in a longitudinal direction. A channel used herein may refer to a path through which a gas (e.g., air or aerosol) passes.

For example, the second segment formed with the crimped polymer sheet may be formed of a material having a thickness between about 5 µm and about 300 µm, for example, between about 10 µm and about 250 µm. In addition, the total surface area of the second segment may be between about 300 square millimeters per millimeter (mm²/mm) and about 1000 mm²/mm. Further, an aerosol cooling element may be formed from a material having a specific surface area between about 10 square millimeters per milligram (mm²/mg) and about 100 mm²/mg.

Meanwhile, the second segment may include a thread containing a volatile flavor ingredient. The volatile flavor ingredient may be menthol. However, embodiments are not limited thereto. For example, the thread may be filled with a sufficient amount of menthol to provide at least 1.5 milligrams (mg) of menthol to the second segment.

A third segment of the filter rod 22 may be a cellulose acetate filter. A desirable length of the third segment may be adopted from a range of 4 mm to 20 mm. For example, the length of the third segment may be about 12 mm. However, embodiments are not limited thereto.

The third segment may be manufactured such that a flavor is generated by spraying a flavoring liquid onto the third segment in a process of manufacturing the third segment. Alternatively, a separate fiber to which the flavoring liquid is applied may be inserted into the third segment. An aerosol generated in the tobacco rod 21 may be cooled as it passes through the second segment of the filter rod 22, and the cooled aerosol may pass through the third segment into the user. Accordingly, when a flavoring element is added to the third segment, the durability of the flavor to be carried to the user may be enhanced.

In addition, the filter rod 22 may include at least one capsule 23. The capsule 23 may perform a function of generating a flavor, or a function of generating an aerosol. For example, the capsule 23 may be of a structure in which a liquid containing a fragrance is wrapped with a film. The capsule 23 may have a spherical or cylindrical shape. However, embodiments are not limited thereto.

Referring to FIG. 6, a cigarette 3 may further include a front end plug 33. The front end plug 33 may be disposed on one side of a tobacco rod 31 opposite to a filter rod 32. The front end plug 33 may prevent the tobacco rod 31 from escaping to the outside, and may also prevent the aerosol liquefied from the tobacco rod 31 during smoking from flowing into an aerosol generating device (e.g., the aerosol generating device 1 of FIGS. 2 to 4).

The filter rod 32 may include a first segment 32a and a second segment 32b. The first segment 32a may correspond to the first segment of the filter rod 22 of FIG. 5, and the second segment 32b may correspond to the third segment of the filter rod 22 of FIG. 5.

The diameter and the total length of the cigarette 3 may correspond to the diameter and the total length of the cigarette 2 of FIG. 5. For example, the length of the front end plug 33 may be about 7 mm, the length of the tobacco rod 31 may be about 15 mm, the length of the first segment 32a may be about 12 mm, and the length of the second segment 32b may be about 14 mm. However, embodiments are not limited thereto.

The cigarette 3 may be wrapped with at least one wrapper 35. The wrapper 35 may have at least one hole through which external air flows inside or internal gas flows outside. For example, the front end plug 33 may be wrapped with a first wrapper 35a, the tobacco rod 31 may be wrapped with a second wrapper 35b, the first segment 32a may be wrapped with a third wrapper 35c, and the second segment 32b may be wrapped with a fourth wrapper 35d. In addition, the cigarette 3 may be entirely wrapped again with a fifth wrapper 35e.

In addition, at least one perforation 36 may be formed on the fifth wrapper 35e. For example, the perforation 36 may be formed in an area surrounding the tobacco rod 31. However, embodiments are not limited thereto. The perforation 36 may perform a function of transferring heat generated by the heater 13 shown in FIGS. 3 and 4 to the inside of the tobacco rod 31.

In addition, the second segment 32b may include at least one capsule 34. The capsule 34 may perform a function of generating a flavor or a function of generating an aerosol. For example, the capsule 34 may have a structure in which a liquid containing a fragrance is wrapped with a film. The capsule 34 may have a spherical or cylindrical shape. However, embodiments are not limited thereto.

The first wrapper 35a may be a combination of general filter wrapping paper and a metal foil such as an aluminum foil. For example, the total thickness of the first wrapper 35a may be in a range of 45 µm to 55 µm, and may be desirably 50.3 µm. In addition, the thickness of the metal foil of the first wrapper 35a may be in a range of 6 µm to 7 µm, and may be desirably 6.3 µm. In addition, the basis weight of the first wrapper 35a may be in a range of 50 g/m² to 55 g/m², and may be desirably 53 g/m².

The second wrapper 35b and the third wrapper 35c may be formed with general filter wrapping paper. The second wrapper 35b and the third wrapper 35c may each be, for example, porous wrapping paper or non-porous wrapping paper.

For example, the porosity of the second wrapper 35b may be 35000 CU. However, embodiments are not limited thereto. In addition, the thickness of the second wrapper 35b may be in a range of 70 µm to 80 µm, and may be desirably 78 µm. In addition, the basis weight of the second wrapper 35b may be in a range of 20 g/m² to 25 g/m², and may be desirably 23.5 g/m².

For example, the porosity of the third wrapper 35c may be 24000CU. However, embodiments are not limited thereto. In addition, the thickness of the third wrapper 35c may be in a range of 60 µm to 70 µm, and may be desirably 68 µm. In addition, the basis weight of the third wrapper 35c may be in a range of 20 g/m² to 25 g/m², and may be desirably 21 g/m².

The fourth wrapper 35d may be formed with polylactic acid (PLA) laminated paper. The PLA laminated paper may refer to three-ply paper including a paper layer, a PLA layer, and a paper layer. For example, the thickness of the fourth wrapper 35d may be in a range of 100 µm to 120 µm, and may be desirably 110 µm. In addition, the basis weight of the fourth wrapper 35d may be in a range of 80 g/m2 to 100 g/m², and may be desirably 88 g/m2.

The fifth wrapper 35e may be formed of sterile paper (e.g., MFW). Here, the sterile paper (MFW) may refer to paper specially prepared such that it has enhanced tensile strength, water resistance, smoothness, or the like, compared to general paper. For example, the basis weight of the fifth wrapper 35e may be in a range of 57 g/m² to 63 g/m², and may be desirably 60 g/m². In addition, the thickness of the fifth wrapper 35e may be in a range of 64 µm to 70 µm, and may be desirably 67 µm.

The fifth wrapper 35e may have a predetermined material internally added thereto. The material may be, for example, silicon. However, embodiments are not limited thereto. Silicon may have properties, such as, for example, heat resistance which is characterized by less change by temperature, oxidation resistance which refers to resistance to oxidation, resistance to various chemicals, water repellency against water, or electrical insulation. However, silicon may not be necessarily used, but any material having such properties described above may be applied (or coated) to the fifth wrapper 35e without limitation.

Although the wrappers 35a, 35b, 35c, 35d, and 35e have been described with reference to FIG. 6, the materials or shapes of the wrappers 35a, 35b, 35c, 35d, and 35e are not limited to the described embodiment, and may include various materials or shapes surrounding the front end plug, the tobacco rod 31, or the filter rod 32.

The front end plug 33 may be formed of cellulose acetate. For example, the front end plug 33 may be manufactured by adding a plasticizer (e.g., triacetin) to cellulose acetate tow. The mono denier of a filament constituting the cellulose acetate tow may be in a range of 1.0 to 10.0, and may be desirably in a range of 4.0 to 6.0. The mono denier of the filament of the front end plug 33 may be more desirably 5.0. In addition, a cross section of the filament constituting the front end plug 33 may be Y-shaped. The total denier of the front end plug 33 may be in a range of 20000 to 30000, and may be desirably in a range of 25000 to 30000. The total denier of the front end plug 33 may be more desirably 28000.

In addition, as needed, the front end plug 33 may include at least one channel, and a cross-sectional shape of the channel may be provided in various ways.

The tobacco rod 31 may correspond to the tobacco rod 21 described above with reference to FIG. 5. Thus, a detailed description of the tobacco rod 31 will be omitted here.

The first segment 32a may be formed of cellulose acetate. For example, the first segment may be a tubular structure including a hollow therein. The first segment 32a may be manufactured by adding a plasticizer (e.g., triacetin) to cellulose acetate tow. For example, the mono denier and the total denier of the first segment 32a may be the same as the mono denier and the total denier of the front end plug 33.

The second segment 32b may be formed of cellulose acetate. The mono denier of a filament constituting the second segment 32b may be in a range of 1.0 to 10.0, and may be desirably in a range of 8.0 to 10.0. The mono denier of the filament of the second segment 32b may be more desirably 9.0. In addition, a cross section of the filament of the second segment 32b may be Y-shaped. The total denier of the second segment 32b may be in a range of 20000 to 30000, and may be desirably 25000.

FIG. 7 is a block diagram illustrating an aerosol generating device according to another example.

According to an embodiment, an aerosol generating device 8 may include a housing 80, a sensing unit 81, a solid-type cartridge (or liquid-type cartridge) 82, a coil 83, and a battery 84. The aerosol generating device 8 may operate in a different manner from that of the aerosol generating device 1 described above with reference to FIGS. 2 to 6. For example, the coil 83 may generate heat by receiving energy from the battery 84. The replaceable cartridge 82 may generate an aerosol using the heat generated by the coil 83. A user may inhale the generated aerosol through an opening 81.

According to an embodiment, the description of the sensing unit 81 may be replaced with the description of the sensing unit 10 provided above with reference to FIGS. 2 to 4.

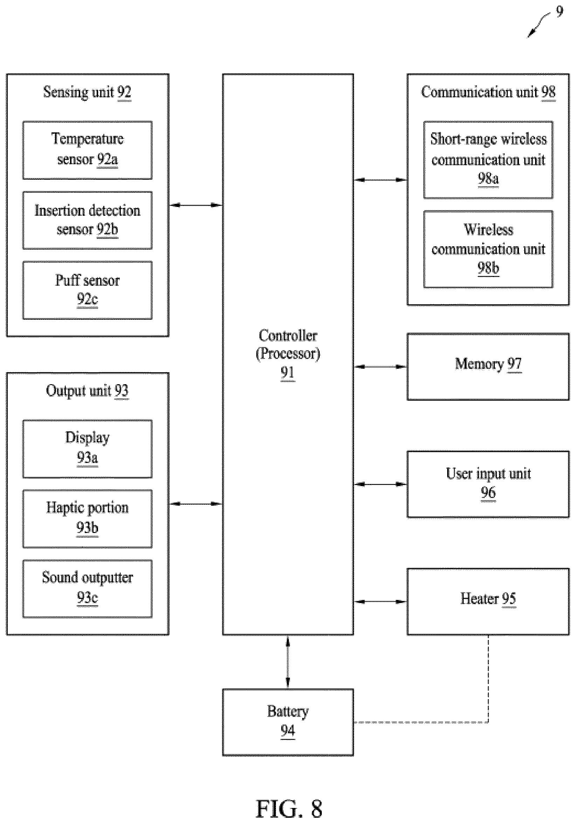

FIG. 8 is a block diagram illustrating an aerosol generating device according to still another example.

According to an embodiment, an aerosol generating device 9 may include a controller 91, a sensing unit 92, an output unit 93, a battery 94, a heater 95, a user input unit 96, a memory 97, and a communication unit 98. However, the internal structure of the aerosol generating device 9 is not limited to what is shown in FIG. 8. It is to be understood by those having ordinary skill in the art to which the disclosure pertains that some of the components shown in FIG. 8 may be omitted or new components may be added according to the design of the aerosol generating device 9.

The sensing unit 92 may sense a state of the aerosol generating device 9 or a state of an environment around the aerosol generating device 9, and transmit sensing information obtained through the sensing to the controller 91. Based on the sensing information, the controller 91 may control the aerosol generating device 9 to control operations of the heater 95, restrict smoking, determine whether an aerosol generating article (e.g., a cigarette, a cartridge, etc.) is inserted, display a notification, and perform other functions.

The sensing unit 92 may include at least one of a temperature sensor 92a, an insertion detection sensor 92b, or a puff sensor 92c. However, embodiments are not limited thereto. For example, the sensing unit 92 may include the sensors of the sensing unit 10 or the sensing unit 81 described above with reference to FIGS. 2 to 8.

The temperature sensor 92a may sense a temperature at which the heater 95 (or an aerosol generating material) is heated. The aerosol generating device 9 may include a separate temperature sensor for sensing a temperature of the heater 95, or the heater 95 itself may perform a function as a temperature sensor. Alternatively, the temperature sensor 92a may be arranged around the battery 94 to monitor a temperature of the battery 94.

The insertion detection sensor 92b may sense whether the aerosol generating article is inserted or removed. The insertion detection sensor 92b may include, for example, at least one of a film sensor, a pressure sensor, a light sensor, a resistive sensor, a capacitive sensor, an inductive sensor, or an infrared sensor, which may sense a signal change by the insertion or removal of the aerosol generating article.

The puff sensor 92c may sense a puff from a user based on various physical changes in an airflow path or airflow channel. For example, the puff sensor 92c may sense the puff based on any one of a temperature change, a flow change, a voltage change, and a pressure change.

The sensing unit 92 may further include at least one of a temperature/humidity sensor, a barometric pressure sensor, a magnetic sensor, an acceleration sensor, a gyroscope sensor, a position sensor (e.g., a global positioning system (GPS)), a proximity sensor, or a red, green, blue (RGB) sensor (e.g., an illuminance sensor), in addition to the sensors 92a through 92c described above. A function of each sensor may be intuitively inferable from its name by those having ordinary skill in the art, and thus a more detailed description thereof will be omitted here.

The output unit 93 may output information about the state of the aerosol generating device 9 and provide the information to the user. The output unit 93 may include at least one of a display 93a, a haptic portion 93b, or a sound outputter 93c. However, embodiments are not limited thereto. When the display 93a and a touchpad are provided in a layered structure to form a touchscreen, the display 93a may be used as an input device in addition to an output device. For example, a user (e.g., the seller 120 or the user 130 of FIG. 1) may generate a design for an article through the display 93a or input an evaluation of a design for an article uploaded to the server 110 by another user.

The display 93a may visually provide the information about the aerosol generating device 9 to the user. The information about the aerosol generating device 9 may include, for example, a charging/discharging state of the battery 94 of the aerosol generating device 9, a preheating state of the heater 95, an insertion/removal state of the aerosol generating article, a limited usage state (e.g., an abnormal article detected) of the aerosol generating device 9, or the like, and the display 93a may externally output the information. As another example, the display 93a may output a graphical user interface (GUI) for generating a design for an article or a GUI for evaluating the design for the article. The display 93a may be, for example, a liquid-crystal display panel (LCD), an organic light-emitting display panel (OLED), or the like. The display 93a may also be in the form of a light-emitting diode (LED) device.

The haptic portion 93b may provide the information about the aerosol generating device 9 to the user in a haptic way by converting an electrical signal into a mechanical stimulus or an electrical stimulus. The haptic portion 93b may include, for example, a motor, a piezoelectric element, or an electrical stimulation device.

The sound outputter 93c may provide the information about the aerosol generating device 9 to the user in an auditory way. For example, the sound outputter 93c may convert an electrical signal into a sound signal and externally output the sound signal.

The battery 94 may supply power to be used to operate the aerosol generating device 9. The battery 94 may supply power to heat the heater 95. In addition, the battery 94 may supply power required for operations of the other components (e.g., the sensing unit 92, the output unit 93, the user input unit 96, the memory 97, and the communication unit 98) included in the aerosol generating device 9. The battery 94 may be a rechargeable battery or a disposable battery. The battery 94 may be, for example, a lithium polymer (LiPoly) battery. However, embodiments are not limited thereto.

The heater 95 may receive power from the battery 94 to heat the aerosol generating material. Although not shown in FIG. 8, the aerosol generating device 9 may further include a power conversion circuit (e.g., a direct current (DC)-to-DC (DC/DC) converter) that converts power of the battery 94 and supplies the power to the heater 95. In addition, when the aerosol generating device 9 generates an aerosol in an induction heating manner, the aerosol generating device 9 may further include a DC-to-alternating current (AC) (DC/AC) converter that converts DC power of the battery 94 into AC power.

The controller 91, the sensing unit 92, the output unit 93, the user input unit 96, the memory 97, and the communication unit 91 may receive power from the battery 94 to perform functions. Although not shown in FIG. 6, a power conversion circuit, for example, a low dropout (LDO) circuit or a voltage regulator circuit, that converts power of the battery 94 and supplies the power to respective components, may further be included.

In an embodiment, the heater 95 may be formed of an electrically resistive material that is suitable. The electrically resistive material may be a metal or a metal alloy including, for example, titanium, zirconium, tantalum, platinum, nickel, cobalt, chromium, hafnium, niobium, molybdenum, tungsten, tin, gallium, manganese, iron, copper, stainless steel, nichrome, or the like. However, embodiments are not limited thereto. In addition, the heater 95 may be implemented as a metal heating wire, a metal heating plate on which an electrically conductive track is arranged, a ceramic heating element, or the like. However, embodiments are not limited thereto.

In another embodiment, the heater 95 may be an induction heater. For example, the heater 95 may include a susceptor that heats the aerosol generating material by generating heat through a magnetic field applied by a coil.

In an embodiment, the heater 95 may include a plurality of heaters. For example, the heater 95 may include a first heater for heating a cigarette and a second heater for heating a liquid.

The user input unit 96 may receive information input from the user or may output information to the user. For example, the user input unit 96 may include a keypad, a dome switch, a touchpad (e.g., a contact capacitive type, a pressure resistive film type, an infrared sensing type, a surface ultrasonic conduction type, an integral tension measurement type, a piezo effect method, etc.), a jog wheel, a jog switch, or the like. However, embodiments are not limited thereto. In addition, although not shown in FIG. 8, the aerosol generating device 9 may further include a connection interface such as a USB interface, and may be connected to another external device through the connection interface such as a USB interface to transmit and receive information or to charge the battery 94.

The memory 97, which is hardware for storing various pieces of data processed in the aerosol generating device 9, may store data processed by the controller 91 and data to be processed thereby. The memory 97 may include at least one type of storage medium of a flash memory type memory, a hard disk type memory, a multimedia card micro type memory, a card type memory (e.g., an SD or XE memory), a random access memory (RAM), a static random access memory (SRAM), a read-only memory (ROM), an electrically erasable programmable read-only memory (EEPROM), a programmable read-only memory (PROM), a magnetic memory, a magnetic disk, or an optical disk. The memory 97 may store an operating time of the aerosol generating device 9, a maximum number of puffs, a current number of puffs, at least one temperature profile, data associated with a smoking pattern of the user, or the like.

The communication unit 98 may include at least one component for communicating with another electronic device. For example, the communication unit 98 may include a short-range wireless communication unit 98a and a wireless communication unit 98b.

The short-range wireless communication unit 98a may include a Bluetooth communication unit, a BLE communication unit, a near field communication unit, a WLAN (Wi-Fi) communication unit, a ZigBee communication unit, an infrared data association (IrDA) communication unit, a Wi-Fi direct (WFD) communication unit, an ultra-wideband (UWB) communication unit, and an Ant+ communication unit. However, embodiments are not limited thereto.

The wireless communication unit 98b may include, for example, a cellular network communication unit, an Internet communication unit, a computer network (e.g., a local area network (LAN) or a wide-area network (WAN)) communication unit, or the like. However, embodiments are not limited thereto. The wireless communication unit 98b may use subscriber information (e.g., international mobile subscriber identity (IMSI)) to identify and authenticate the aerosol generating device 9 in a communication network.

The controller 91 may control the overall operation of the aerosol generating device 9. In an embodiment, the controller 91 may include at least one processor. The at least one processor may be implemented as an array of a plurality of logic gates, or may be implemented as a combination of a general-purpose microprocessor and a memory in which a program executable by the microprocessor is stored. In addition, it is to be understood by those having ordinary skill in the art to which the present disclosure pertains that it may be implemented in other types of hardware.

The controller 91 may control the temperature of the heater 95 by controlling the supply of power from the battery 94 to the heater 95. For example, the controller 91 may control the supply of power by controlling switching of a switching element between the battery 94 and the heater 95. For another example, a direct heating circuit may control the supply of power to the heater 95 according to a control command from the controller 91.

The controller 91 may analyze a sensing result obtained by the sensing of the sensing unit 92 and control processes to be performed thereafter. For example, the controller 91 may control power to be supplied to the heater 95 to start or end an operation of the heater 95 based on the sensing result obtained by the sensing unit 92. For another example, the controller 91 may control an amount of power to be supplied to the heater 95 and a time for which the power is to be supplied, such that the heater 95 may be heated up to a predetermined temperature or maintained at a desired temperature, based on the sensing result of the sensing unit 92.

The controller 91 may control the output unit 93 based on the sensing result of the sensing unit 92. For example, when the number of puffs counted through the puff sensor 92c reaches a preset number, the controller 91 may inform the user that the aerosol generating device 9 is to be ended soon, through at least one of the display 93a, the haptic portion 93b, or the sound outputter 93c.

In an embodiment, the controller 91 may control a power supply time and/or a power supply amount for the heater 95 according to a state of the aerosol generating article sensed by the sensing unit 92. For example, when an aerosol generating material is in an over-humidified state, the controller 91 may control the power supply time for an inductive coil to increase a preheating time, compared to a case where the aerosol generating material is in a general state.

FIG. 9 is a diagram illustrating a configuration of a server according to an embodiment.

A server 900 includes a communication unit 910, a processor 920, and a memory 930. For example, the server 900 may be the server 110 described above with reference to FIG. 1.

The communication unit 910 may be connected to the processor 920 and the memory 930 and transmit and receive data to and from the processor 920 and the memory 930. The communication unit 910 may be connected to another external device and transmit and receive data to and from the external device. Hereinafter, transmitting and receiving "A" may refer to transmitting and receiving "information or data indicating A".

The communication unit 910 may be implemented as a circuitry in the server 900. For example, the communication unit 910 may include an internal bus and an external bus. In another example, the communication unit 910 may be an element that connects the server 900 and the external device. The communication unit 910 may be an interface. The communication unit 910 may receive data from the external device and transmit the data to the processor 920 and the memory 930.

The processor 920 may process the data received by the communication unit 910 and data stored in the memory 930. A "processor" may be a hardware-implemented data processing device having a physically structured circuit to execute desired operations. The desired operations may include, for example, codes or instructions included in a program. The hardware-implemented data processing device may include, for example, a microprocessor, a central processing unit (CPU), a processor core, a multi-core processor, a multiprocessor, an application-specific integrated circuit (ASIC), and a field-programmable gate array (FPGA).

The processor 920 may execute computer-readable code (e.g., software) stored in a memory (e.g., the memory 930) and instructions triggered by the processor 920.

The memory 930 may store therein the data received by the communication unit 910 and the data processed by the processor 920. For example, the memory 930 may store the program (or an application, or software). The program to be stored may be a set of syntaxes that are coded and executable by the processor 920 to manage design information of a tobacco-related article.

According to an aspect, the memory 930 may include, for example, at least one volatile memory, nonvolatile memory, random-access memory (RAM), flash memory, a hard disk drive, and an optical disc drive.

The memory 930 may store an instruction set (e.g., software) for operating the server 900. The instruction set for operating the server 900 may be executed by the processor 920.

FIG. 10 is a flowchart illustrating a method of managing design information of a tobacco-related article according to an embodiment.

Operations 1010 through 1040 may be performed by the server 900 described above with reference to FIG. 9. Before operation 1010 is performed, a user terminal (e.g., the first user terminal 122 or the second user terminal 132) may be connected to the server 900 using wireless communication. Before operation 1010 is performed, a seller (e.g., the seller 120 of FIG. 1) may create a design for a tobacco-related article.

In operation 1010, the server 900 may receive target type information of a tobacco-related target article and target design information of the target article from a first user terminal (e.g., the first user terminal 122 of FIG. 1) of the seller.

For example, the target type information may include information indicating that the tobacco-related target article is a cigarette-type electronic cigarette device or tipping paper of a general cigarette. As another example, the target type information may include information related to a type or a model name of the target article, which is an electronic cigarette device.

For example, the target design information may include a rendered image file of a design of the tobacco-related target article created by the seller, detailed dimensions of the shape of the design, color names or color codes used in the design, and the like. The target design information may further include an additional description of the design of the seller.

In operation 1020, the server 900 may issue a blockchain-based non-fungible token (NFT) containing ownership information indicating the seller is an initial owner of the target design information. For example, the ownership information of the target design information may be stored through a blockchain. In operation 1030, the server 900 may perform a purchase contract of the NFT between the seller and an administrator (or an operating entity) of a server.

According to an embodiment, the operating entity of the server 900 may determine to purchase the target design information from the seller among a plurality of pieces of design information uploaded to the server 900 by evaluating various factors, and the operating entity may purchase an NFT for the target design information from the seller through the server 900.

In an embodiment, the seller may set a desired selling price for an NFT of target design information owned by the seller and determine the number of NFTs to be issued.

In an embodiment, when the administrator of the server 900 purchases the target design information of the seller uploaded to the server 900, the administrator of the server 900 may purchase all or part of the NFTs issued by the seller. For example, the administrator of the server 900 may apply the purchased target design information to an actual article, and sell the actual article to which the target design information is applied.

In operation 1040, the server 900 may store ownership change information of the NFT when the purchase contract of the NFT of the target design information is performed with the seller. Similar to the initially stored ownership information, related information including the ownership change information and sales history of the NFT may be stored in the blockchain. The ownership change information indicating that the administrator of the server 900 has become the owner of the purchased NFT may be stored in the blockchain.

Allowing consumers to directly design tobacco-related articles may promote the development of creative and aesthetic products based on consumer experiences and perspectives and the vitalization of the tobacco industry.

Consumers of tobacco-related articles may directly create profit through NFT trades.

When designs of tobacco-related articles are released in advance, it is possible to monitor popular designs and reduce the time and cost of developing tobacco-related articles by directly applying the designs to products.

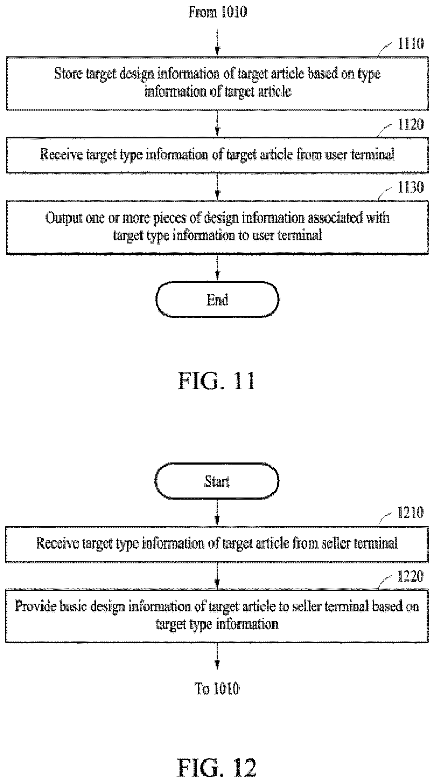

FIG. 11 is a flowchart illustrating a method of providing design information to a user according to an example.

According to an embodiment, operations 1110 to 1130 may be further performed after operation 1010 described above with reference to FIG. 10 is performed.

In operation 1110, the server 900 may store target design information of the target article based on the type information of the target article received from the first user terminal of the seller. For example, design information indicating the type of the target article is a cigar and design information indicating the type of the target article is a cigarette-type electronic cigarette may be classified into different categories and stored separately.

According to an embodiment, the server 900 may provide a function to classify design information according to type information of tobacco-related articles.

In operation 1120, the server 900 may receive target type information of the target article from a second user terminal (e.g., the second user terminal 132) of the user. For example, the user may access the server 900 through the second user terminal, and transmit information indicating the target type is a cigarette-type electronic cigarette device to the server 900 by selecting a cigarette-type electronic cigarette device as the target type information from a menu provided by the server 900.

In operation 1130, the server 900 may output (or output) one or more pieces of design information associated with the target type information received from the second user terminal to the second user terminal. For example, when the user selects a cigarette-type electronic cigarette device as the target type information from the menu provided by the server 900 through the second user terminal, the user may be provided with one or more pieces of design information of the cigarette-type electronic cigarette device.

According to an embodiment, when the user selects specific design information from among the one or more pieces of design information output to the second user terminal, the user may check details of the selected design information. For example, the user may check the design of the article, a trading price of an NFT of the design, or owner information.

FIG. 12 is a flowchart illustrating a method of providing basic design information to a seller according to an example.

According to an embodiment, operations 1210 and 1220 may be performed before operation 1010 described above with reference to FIG. 3 is performed.

In operation 1210, the server 900 may receive target type information of a target article from a first user terminal (e.g., the first user terminal 122 of FIG. 1) of a seller. The seller may select the target article to be designed before creating a design for a tobacco-related article. The target article that the seller wants to design may be an article already on sale or not. For example, if the target article is a new article that is not currently sold on the market, the seller may freely create a design for the article. As another example, if the target article is an article currently on sale on the market, the seller may need all matters necessary for designing, such as the design or specifications of the target article.

According to an embodiment, the seller may transmit target type information such as a model name, a type, a series, or a generation of the target article to be designed to the server 900 through a second seller terminal. For example, the server 900 may provide a plurality of pieces of type information to the seller through a menu, and the seller may select a target article to be designed from among the plurality of pieces of type information.

In operation 1220, the server 900 may provide basic design information of the target article to the first user terminal based on the target type information received from the first user terminal of the seller. For example, when the server 900 receives target type information of "lil Hybrid 2.0" from the first user terminal, the server 900 may provide basic design information of a target article corresponding to "lil Hybrid 2.0" to the first user terminal.

According to an embodiment, the basic design information may include cartridge specifications, the presence/absence, position, shape or dimensions of a display, contents displayed on the display, a battery level display method, the presence/absence, position, shape or dimensions of a button, battery charging port specifications, device specifications or paper composition materials, and dimensions.

According to an embodiment, the basic design information may be provided in the form of a file containing data including design images and size values of the article. For example, the seller may design the target article by using or changing the existing specifications of the article with reference to the basic design information provided to the first user terminal.

According to an embodiment, the basic design information may be provided in a form that allows the user to access a manufacturing tool linked with the server 900 that provides the shape and size values of the article.

According to an embodiment, the basic design information may be provided in a form that allows the server 900 to transmit a design base of an article corresponding to a target type to the first user terminal and allows the user to create a design on his/her own terminal (e.g., the first user terminal or another electronic device) and transmit a completed design to the server 900 through the first user terminal. For example, the design base may include a sketch reflecting the size of the article on the background.

After operation 1220 is performed, in operation 1010 of FIG. 10, the server 900 may receive target type information of a tobacco-related target article and target design information of the target article from the first user terminal. Through operations 1210 and 1220, the seller may accurately and efficiently design the target article based on the basic design information necessary for designing the target article, provided by the server 900.

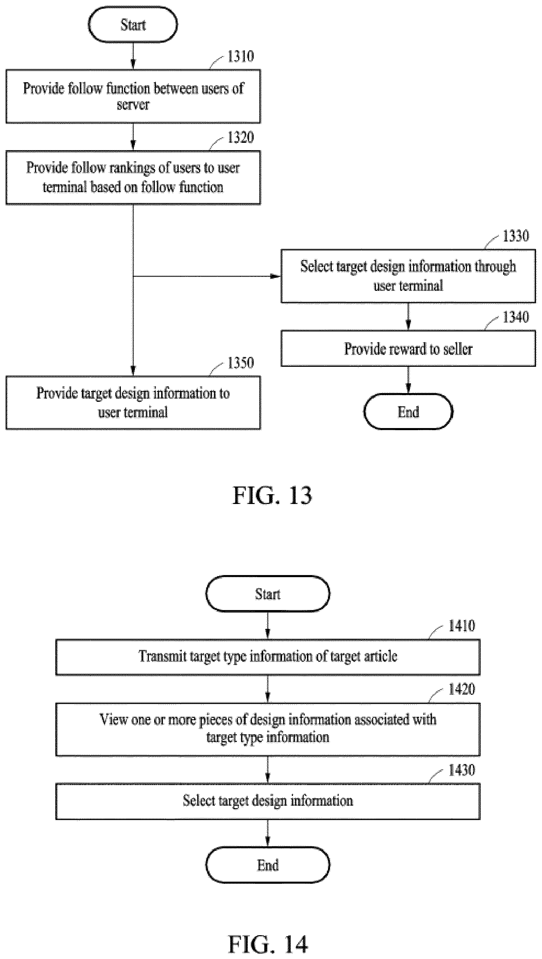

FIG. 13 is a flowchart illustrating a method of selecting design information based on a follow function and providing a reward according to an example.

In operation 1310, the server 900 may provide a follow function between users registered to the server 900. The follow function may include one or more of a user's following another user or a user's following predetermined design information. The server 900 may additionally provide an unfollow function and a block function between the users.

In operation 1320, the server 900 may provide follow rankings of the users to the second user terminal of the user based on the follow function. For example, the follow rankings may include one or more of rankings of the users or rankings of pieces of design information. A user having more followers may have a higher ranking. Alternatively, design information with a higher preference may have a higher ranking. The users may select target design information based on the follow rankings. The administrator of the server 900 may check designs preferred by the users through the follow ranking.

According to an embodiment, the follow rankings may be automatically provided to all users, or may be provided only to users who want.

According to an embodiment, the follow rankings may be classified and provided according to type information of tobacco-related articles.

After operation 1320 is performed, operation 1330 or operation 1350 may be performed. Operations 1330 and 1350 may be performed independently and in parallel.

In operation 1330, the user selects target design information through the second user terminal. For example, "selecting" in operation 1330 may be feedback from the user by which the popularity of the design can be measured. For example, the user may select the target design information to show "like" or vote for a preference for the design.

According to an embodiment, rankings of target designs selected by a number of users may be provided.

In operation 1340, the server 900 may provide a reward to the seller who creates the selected design information. For example, the reward may be points for increasing the ranking. As another example, the reward may be money, or mileage or points that can be used in a platform of the server 900. According to an embodiment, the server 900 may provide a reward for a target design selected by a number of users.

According to an embodiment, the server 900 may provide a limited edition of a tobacco-related product or a purchase opportunity as a reward to a seller of a popular design.

After operation 1320 is performed, in operation 1350, the server 900 may provide target design information to the second user terminal of the user. When the server 900 provides the follow rankings of the users to the second user terminal, the user may select target design information to be viewed based on the follow rankings, and the server 900 may provide the target design information to the second user terminal such that the user may view the selected target design information.

FIG. 14 is a flowchart illustrating a method of selecting design information by a user according to an example.

Operations 1410 to 1430 may be performed by a second user terminal (e.g., the second user terminal 132 of FIG. 1) of a user.

In operation 1410, the second user terminal may transmit target type information of a target article to a server. The user may transmit the target type information to the server 900 through the second user terminal based on a classification function according to type information of articles provided by the server 900.

In operation 1420, the user may view one or more pieces of design information associated with the target type information through the second user terminal. For example, the user may view one or more pieces of design information associated with a liquid-type electronic cigarette device.

In operation 1430, the user may select target design information from among the one or more pieces of design information through the second user terminal.

According to an embodiment, the user may view a detailed number of NFTs on sale, price, and ownership information of the selected design through the second user terminal.

According to an embodiment, the user may purchase all or part of the NFTs on sale of the selected design through the second user terminal. For example, when the user purchases a portion of the NFTs and then, a purchase contract of the portion of the NFTs is performed with the administrator of the server 900, each of the two or more users including the seller who has the portion of the NFTs may receive incentives according to their shares of the NFTs for sales profit produced by the commercialization of the design in the future.

According to an embodiment, the user may reset the price of the NFT purchased for the target design information and resell the NFT in the future. The server 900 may provide a platform for NFT trades between users.

The units described herein may be implemented using a hardware component, a software component and/or a combination thereof. A processing device may be implemented using one or more general-purpose or special-purpose computers, such as, for example, a processor, a controller and an arithmetic logic unit (ALU), a digital signal processor (DSP), a microcomputer, a field-programmable gate array (FPGA), a programmable logic unit (PLU), a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciate that a processing device may include multiple processing elements and multiple types of processing elements. For example, the processing device may include a plurality of processors, or a single processor and a single controller. In addition, different processing configurations are possible, such as parallel processors.

The software may include a computer program, a piece of code, an instruction, or some combination thereof, to independently or uniformly instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network-coupled computer systems so that the software is stored and executed in a distributed fashion. The software and data may be stored by one or more non-transitory computer-readable recording mediums.

The methods according to the above-described embodiments may be recorded in non-transitory computer-readable media including program instructions to implement various operations of the above-described embodiments. The media may also include, alone or in combination with the program instructions, data files, data structures, and the like. The program instructions recorded on the media may be those specially designed and constructed for the purposes of embodiments, or they may be of the kind well-known and available to those having skill in the computer software arts. Examples of non-transitory computer-readable media include magnetic media such as hard disks, floppy disks, and magnetic tape; optical media such as CD-ROM discs, DVDs, and/or Blue-ray discs; magneto-optical media such as optical discs; and hardware devices that are specially configured to store and perform program instructions, such as read-only memory (ROM), random access memory (RAM), flash memory (e.g., USB flash drives, memory cards, memory sticks, etc.), and the like. Examples of program instructions include both machine code, such as produced by a compiler, and files containing higher-level code that may be executed by the computer using an interpreter.

The above-described devices may be configured to act as one or more software modules in order to perform the operations of the above-described embodiments, or vice versa.

A number of embodiments have been described above. Nevertheless, it should be understood that various modifications may be made to these embodiments. For example, suitable results may be achieved if the described techniques are performed in a different order and/or if components in a described system, architecture, device, or circuit are combined in a different manner and/or replaced or supplemented by other components or their equivalents.

Accordingly, other implementations are within the scope of the following claims.

## Claims

1. A method of managing design information of a tobacco-related article, performed by a server, the method comprising:
receiving target type information of a tobacco-related target article and target design information of the target article from a seller terminal;
issuing a blockchain-based non-fungible token (NFT) containing ownership information indicating a seller of the seller terminal is an initial owner of the target design information;
performing a purchase contract of the NFT between the seller and an administrator of the server; and
storing ownership change information of the NFT when the purchase contract is performed.

2. The method of claim 1, further comprising:
storing the target design information of the target article based on the type information of the target article;
receiving the target type information of the target article from a user terminal; and
outputting one or more pieces of design information associated with the target type information to the user terminal.

3. The method of claim 1, further comprising:
receiving the target type information of the target article from the seller terminal;
providing basic design information of the target article to the seller terminal based on the target type information.

4. The method of claim 1, further comprising:
providing a follow function between users of the server; and
providing follow rankings of the users to a user terminal based on the follow function.

5. The method of claim 4, further comprising:
providing a reward to the seller when the user terminal selects the target design information.

6. A server for performing a method of managing design information of a tobacco-related article, the server comprising:
a memory configured to store a program for managing design information; and
a processor configured to execute the program,
wherein the processor is configured to perform:
receiving target type information of a tobacco-related target article and target design information of the target article from a seller terminal;
issuing a blockchain-based non-fungible token (NFT) containing ownership information indicating a seller of the seller terminal is an initial owner of the target design information;
performing a purchase contract of the NFT between the seller and an administrator of the server; and
storing ownership change information of the NFT when the purchase contract is performed.

7. The server of claim 6, wherein
the program is further configured to perform:
storing the target design information of the target article based on the type information of the target article;
receiving the target type information of the target article from a user terminal; and
outputting one or more pieces of design information associated with the target type information to the user terminal.

8. The server of claim 6, wherein the program is further configured to perform:
receiving the target type information of the target article from the seller terminal; and
providing basic design information of the target article to the seller terminal based on the target type information.

9. The server of claim 5, wherein the program is further configured to perform:
providing a follow function between users of the server; and
providing follow rankings of the users to a user terminal based on the follow function.

10. The server of claim 9, wherein the server is further configured to perform providing a reward to the seller when the user terminal selects the target design information.
